# EUROPEAN PATENT SPECIFICATION

(11) **EP 3 811 854 B1**
(45) Date of publication and mention of the grant of the patent: **13.12.2023**
(21) Application number: 20202287.7
(22) Date of filing: 16.10.2020
(51) Int. Cl.: A61B 5/00, A61B 5/0205, A61B 5/021, A61B 5/024, A61B 5/053, A61B 5/11, G06F 3/044, G16H 40/63, A61B 5/1455, A61B 5/0245, G06F 1/16

(54) **WEARING DETECTION METHOD, CHIP AND DEVICE**
VERFAHREN ZUR ERKENNUNG, OB EINE VORRICHTUNG GETRAGEN WIRD, CHIP UND VORRICHTUNG
PROCÉDÉ DE DÉTECTION DE PORTAGE D'UN DISPOSITIF, PUCE ET DISPOSITIF

(30) Priority: 21.10.2019 CN 201911002363
(43) Date of publication of application: 28.04.2021
(73) Proprietor: SHENZHEN GOODIX TECHNOLOGY CO., LTD., Shenzhen, Guangdong 518045 (CN)
(72) Inventor: LIU, Chang, Shenzhen, Guangdong 518045 (CN); YANG, Wangwang, Shenzhen, Guangdong 518045 (CN); DENG, Fujian, Shenzhen, Guangdong 518045 (CN)
(74) Representative: Elzaburu S.L.P.

(56) References cited:
- EP-A1- 3 506 052
- WO-A1-2019/107741
- CN-A- 107 106 044
- CN-A- 107 817 537
- CN-A- 108 564 179
- US-A1- 2010 076 331
- US-A1- 2016 154 952

## Description

### Technical field

The present application relates to the field of wearable device technologies and, in particular, to a wearing detection method, an apparatus, a chip, a device and a storage medium.

### Background

With the development of intelligent devices, the intelligent devices have more and more functions. In order to detect health status of a human body, it is feasible to use a sensor (e.g., a sensor disposed close to the outer surface of the human body, a sensor approaching above the outer surface of the human body, or a sensor approaching the outer surface of the human body in other ways) in an intelligent device (e.g., a wearable device) for detection. The wearable device can not only monitor health status of a wearer for a long time, but also allow the wearer to normally carry out daily activities, travel, go on and off duty or participate in other activities. The health status of the human body monitored by this kind of wearable device may include heart rate, blood oxygen saturation, activity level, blood pressure, galvanic skin response or other information about the wearer's body.

It is necessary to judge a worn state of a wearable device when the wearable device monitors health status of a human body, so as to ensure that a user's health status is detected on the basis of the wearable device being in the worn state. And wearing quality may affect accuracy of detection results. In the prior art, the worn state of the wearable device is usually detected by using optical wearing detection.

However, in the prior art, when the optical wearing detection is configured to detect the worn state of the wearable device, it is impossible to distinguish between the human body and an object, leading to inaccuracy of the detection results.

WO2019107741A1 discloses a device, including: a capacitive sensor, a heart rate sensor including at least one light-emitting unit, a first light receiving unit, and a second light receiving unit; and a processor.

US20160154952A1 discloses a method, involving: measuring, based on output of a capacitive sensor, a capacitance value indicative of proximity of the wearable device to a user; detecting a change in the capacitance value within a defined time interval, the change being greater than or equal to a threshold change indicative of the wearable device not being proximate to the user's skin; determining that the wearable device has been removed from the user in response to detecting that the change in the capacitance value within the defined time interval is greater than or equal to the threshold change. CN107817537A discloses a wear detecton control method and device for a wearable device.

### Summary

The present application provides a wearing detection method according to claim **1,** a chip according to claim **7,** and a device according to claim **8,** with further embodiments defined by dependent claims **2-6, 9-10,** so as to detect a worn state of a wearable device, which improves accuracy of worn state detection.

### Brief description of the drawings

In order to more clearly illustrate technical solutions in embodiments of the present application or in the prior art, the drawings used in describing the embodiments or the prior art are briefly described below. Obviously, the drawings described below are some examples of the present application, and those of ordinary skill in the art could obtain other drawings from these drawings without creative effort.
FIG. 1 is a diagram of an exemplary application scenario of a technical solution of the present application;
FIG. 2 is a schematic flowchart of a wearing detection method provided by an embodiment of the present application;
FIG. 3 is a schematic flowchart of a wearing detection method provided by another embodiment of the present application;
FIG. 4 is a schematic flowchart of a wearing detection method provided by yet another embodiment of the present application;
FIG. 5 is a schematic structural diagram of a wearing detection apparatus provided by an embodiment of the present application;
FIG. 6 is a schematic structural diagram of a device provided by an embodiment of the present application.

### Detailed description of the embodiments

The terms "first", "second", "third", "fourth" and the like (if present) in the description, claims and the above drawings of the present application are used to distinguish similar objects rather than to describe a specific sequence or order. It should be understood that the terms used in this way may be interchanged in suitable situations, such that the embodiments of the present application described herein can be implemented in a sequence other than those illustrated or described herein. In addition, the terms "include" and "have" and any variations of them are intended to cover a non-exclusive inclusion. For example, processes, methods, systems, products, or devices that include a series of steps or units are not necessarily limited to those steps or units clearly listed, but may include other steps or units that are not clearly listed or inherent to such processes, methods, products or devices. The term "a plurality of" shown in the embodiments of the present application refers to two or more.

With the development of intelligent devices, the intelligent device has more and more functions. In order to detect health status of a human body, it is feasible to use a sensor (e.g., a sensor disposed close to the outer surface of the human body, a sensor approaching above the outer surface of the human body, or a sensor approaching the outer surface of the human body in other ways) in an intelligent device (e.g., a wearable device) for detection. The wearable device can not only monitor health status of a wearer for a long time, but also allow the wearer to carry out daily activities, travel, go on and off duty or participate in other activities. The health status of the human body monitored by this kind of wearable device may include heart rate, blood oxygen saturation, activity level, blood pressure, galvanic skin response or other information about the wearer's body. It is necessary to judge a worn state of a wearable device when the wearable device monitors health status of a human body, so as to ensure that a user's health status is detected on the basis of the wearable device being in the worn state. And wearing quality may affect accuracy of detection results. However, in the prior art, when optical wearing detection is configured to detect the worn state of the wearable device, it is impossible to distinguish between the human body and an object, leading to inaccuracy of the detection results. In order to solve the above problem, the present application provides a wearing detection method, an apparatus, a chip, a device and a storage medium.

An exemplary scenario of embodiments of the present application is introduced below.

Wearing detection methods provided by the embodiments of the present application can be implemented by wearing detection apparatuses provided by the embodiments of the present application. The wearing detection apparatus may be part or the whole of a terminal device. The terminal device may be a wearable device, such as a heart rate bracelet, a heart rate earphone, a health bracelet, a health watch, a blood oxygen bracelet, a blood oxygen watch and other optical wearable devices; and the terminal device may also be a medical device, a fitness device, etc. The apparatus may also be a sensor, a chip or others in the terminal device, which is not limited in the embodiments of the present application.

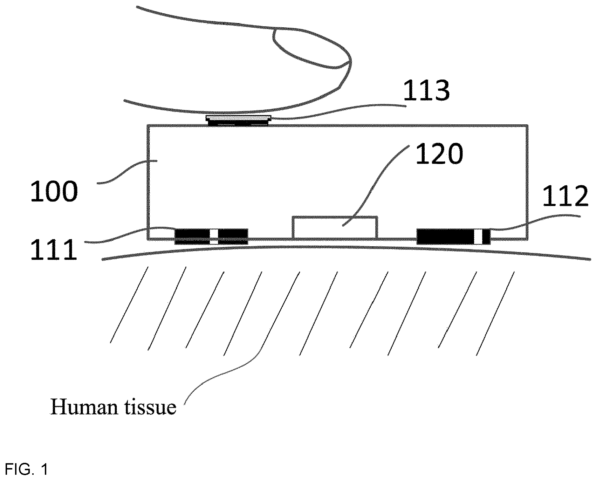

A wearable device is taken as an example of the wearing detection apparatus provided by the embodiments of the present application for description below. FIG. 1 is a diagram of an exemplary application scenario of a technical solution of the present application. As shown in FIG. 1, a wearable device can include a housing 100, a first electrode 111, a first electrode 112 and at least one optical detection module 120. The wearable device is usually worn on a wrist, an ankle or other parts of a user. The housing 100 is configured to protect the wearable device, and the quantity of the first electrode is not limited to 2. The first electrode 111 and the first electrode 112 are both located at the bottom of the housing, and when the housing is worn on a human body, the first electrode 111 and the first electrode 112 come into contact with human tissue; when the human tissue or other metal objects come into contact with the first electrode 111 and the first electrode 112, capacitance signals of the first electrode 111 and the first electrode 112 will change, and an impedance signal between the first electrode 111 and the first electrode 112 will decrease. Therefore, the impedance signal between the first electrode 111 and the first electrode 112, and/or the capacitance signal of the first electrode 111 and/or the first electrode 112 can be obtained through the first electrode 111 and the first electrode 112, so as to judge whether there is human tissue or metal. The optical detection module 120 includes an optical transmitter and an optical receiver. The optical transmitter may include one or more light emitting diodes. After being reflected, scattered and absorbed by the human tissue, some of light emitted by the light emitting diodes can be emitted from a surface of the human tissue, and received by the optical receiver of the optical detection module 120, where the optical receiver may include one or more photodiodes. The first optical signal can be obtained by detecting the optical receiver, and on the basis that it is judged that there is human tissue or metal, it can be judged through the first optical signal whether what is in contact with the wearable device is the human tissue or not, thereby solving the problem that the optical wearing detection cannot distinguish a human from an object and the problem that electrode wearing detection cannot distinguish a human from metal. In addition, the wearable device provided by the embodiments of the present application can also include at least one second electrode 113, where the second electrode 113 can be provided on the top of the housing 100 or other locations where a user is able to touch; and the second electrode is configured to judge whether there is a user's operation, so as to judge whether a start of an electrocardiograph detection function is required. Based on this, the embodiments of the present application provide a wearing detection method, an apparatus, a chip, a device and a storage medium.

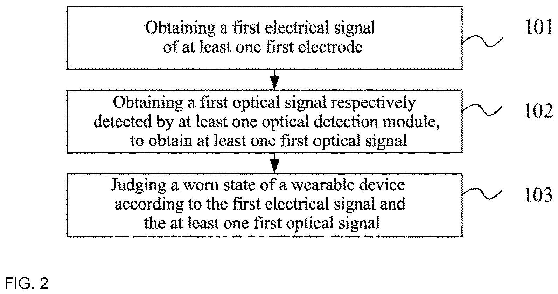

FIG. 2 is a schematic flowchart of a wearing detection method provided by an embodiment of the present application. The method can be executed by a wearing detection apparatus, which can be implemented in the form of software and/or hardware. For instance, the apparatus may be part or the whole of a terminal device. The terminal device may be a wearable device, such as a heart rate bracelet, a heart rate earphone, a health bracelet, a health watch, a blood oxygen bracelet, a blood oxygen watch and other optical wearable devices; and the terminal device may also be a medical device, a fitness device, etc. The apparatus may also be a detection chip or others in the terminal device. The wearing detection method is described below by taking a terminal device as an executive entity. As shown in FIG. 2, the wearing detection method provided by the embodiment of the present application may include:
Step S101: obtaining a first electrical signal of at least one first electrode.

The type of the first electrical signal is an impedance signal according to the invention and is not limited in examples not falling under the scope of the claims, and may be, for instance, a capacitance signal, a reactance signal, a capacitive reactance signal, and so on. The wearable device includes at least one first electrode. If the wearable device includes one first electrode, the first electrical signal can be the capacitance signal (in an embodiment not covered by the scope of the claims) of the first electrode; if the wearable device includes two or more first electrodes, in a possible implementation, in an embodiment not covered by the scope of the claims, the first electrical signal can be the capacitance signal of each of the first electrodes.

According to the invention, the first electrical signal includes at least one impedance signal, and the at least one impedance signal is an impedance signal between any two first electrodes of a plurality of first electrodes.

If the plurality of first electrodes are two first electrodes, one impedance signal is obtained by obtaining an impedance signal between the two first electrodes; if the plurality of first electrodes are three or more first electrodes, at least one impedance signal is obtained by obtaining an impedance signal between any two first electrodes among the plurality of first electrodes or by obtaining an impedance signal between any two first electrodes of a preset quantity among the plurality of first electrodes, which is not limited in the embodiment of the present application.

Step S102: obtaining a first optical signal respectively detected by at least one optical detection module, to obtain at least one first optical signal.

The quantity of the optical detection module may be one or more, which is not limited in the embodiment of the present application. The first optical signal corresponding to each optical detection module can be obtained through the optical detection module. The optical detection module includes an optical transmitter and an optical receiver, and the first optical signal is an optical signal received by the optical receiver after being transmitted by the optical transmitter and going through a human body. The form of the first optical signal is not limited in the embodiment of the present application. For instance, the first optical signal may be an analog signal or a digital signal.

Step S103: judging a worn state of a wearable device according to the first electrical signal and the at least one first optical signal.

The worn state of the wearable device can include being in the worn state and being in a non-worn state. The worn state is graded by judging wearing quality and setting the worn state of the wearable device as failure in wearing, high wearing quality and low wearing quality.

The embodiment of the present application is described by taking an example in which the first electrical signal is at least one impedance signal, then the judging the worn state of the wearable device according to the first electrical signal and the at least one first optical signal includes: judging the worn state of the wearable device according to the at least one impedance signal and the at least one first optical signal. In a possible implementation, the worn state of the wearable device may be judged through one impedance signal and one or more first optical signals, and the worn state of the wearable device may also be judged through a plurality of impedance signals and one or more first optical signals. The embodiment of the present application does not limit the adopted impedance signal and first optical signal, as well as the quantity of the impedance signal and the quantity of the first optical signal. Additionally, the embodiment of the present application does not limit the specific implementation of how to judge the worn state of the wearable device according to at least one impedance signal and at least one first optical signal.

The judging the worn state of the wearable device according to the first electrical signal and the at least one first optical signal includes:
determining an impedance signal, which is within a first preset range, of the at least one impedance signal to be a target impedance signal; determining a first optical signal, which is within a second preset range, of the at least one first optical signal to be a target optical signal; judging that the wearable device is in the worn state, if a quantity of the target impedance signal reaches a first preset threshold and a quantity of the target optical signal reaches a second preset threshold.

By determining the target impedance signal of the at least one impedance signal through setting the first preset range and through judging whether the impedance signal is within the first preset range, the impedance signal that satisfies detecting a human body or metal can be determined, where a smaller impedance signal indicates that a distance between human tissue or metal and the first electrode is smaller, and thus the wearing quality is higher.

By determining the target optical signal of the at least one first optical signal through setting the second preset range and through judging whether the first optical signal is within the second preset range, the target optical signal that satisfies detecting a human body can be determined.

The embodiment of the present application does not limit the specific range of the first preset range and the second preset range, which can be set according to actual requirements. After determining the target impedance signal and the target optical signal, the quantity of the target impedance signal and the quantity of the target optical signal can be judged respectively, so as to judge whether the wearable device is in the worn state. The embodiment of the present application can be implemented by setting the first preset threshold and the second preset threshold, and if the quantity of the target impedance signal reaches the first preset threshold and the quantity of the target optical signal reaches the second preset threshold, it is judged that the wearable device is in the worn state.

The embodiment of the present application does not limit the specific values of the first preset threshold and the second preset threshold. Specifically, the first preset threshold can be set according to the quantity of the first electrode and the second preset threshold can be set according to the quantity of the optical detection module. For instance, if there are only two first electrodes, then the first preset threshold can be set as 1; if there are four first electrodes, then the first preset threshold can be set as 5; if the quantity of the optical detection module is 4, then the second preset threshold can be set as 3, which are not limited in the embodiment of the present application.

In the embodiment of the present application, the worn state of the wearable device is judged by determining, in the at least one impedance signal, the quantity of the impedance signal which is within a preset range and determining, in the at least one first optical signal, the quantity of the first optical signal which is within a preset range, thereby improving reliability of the worn state detection of the wearable device.

In a second possible implementation, the determining the worn state of the wearable device according to the first electrical signal and the at least one first optical signal includes:
determining an impedance signal, which is within a first preset range, of the at least one impedance signal to be a target impedance signal; determining a first optical signal, which complies with a fluctuation rule of a human physiological signal, of the at least one first optical signal to be a target optical signal; judging that the wearable device is in the worn state, if the quantity of the target impedance signal reaches a first preset threshold and the quantity of the target optical signal reaches a second preset threshold.

The difference between the second possible implementation and the first possible implementation is how to determine the target optical signal, and for the other parts, the introduction of the solution for the first possible implementation can be referred to, which will not be repeated here.

If the optical detection module has detected the first optical signal, it can be determined whether there is a fluctuation rule of a human physiological signal in the first optical signal by analyzing the first optical signal. The fluctuation rule of the human physiological signal is configured to distinguish a human body from an object, and the first optical signal with the fluctuation rule of the human physiological signal in the at least one first optical signal is determined as the target optical signal.

In the embodiment of the present application, the worn state of the wearable device is judged by determining, in the at least one impedance signal, the quantity of the impedance signal which is within a preset range and determining, in the at least one first optical signal, the quantity of the first optical signal which complies with the fluctuation rule of the human physiological signal, thereby improving the reliability of the worn state detection of the wearable device.

After it is judged that the wearable device is in the worn state, the wearing detection method provided by the embodiment of the present application can further include: starting a first detection function, and the first detection function includes at least one of the following: a heart rate detection function, a blood oxygen saturation detection function and a blood pressure detection function. The detection function is not limited in the embodiment of the present application. By starting the heart rate detection function, the blood oxygen saturation detection function, the blood pressure detection function and the like on the basis that it is judged that the wearable device is in the worn state, not only can power be saved, but also user experience can be improved.

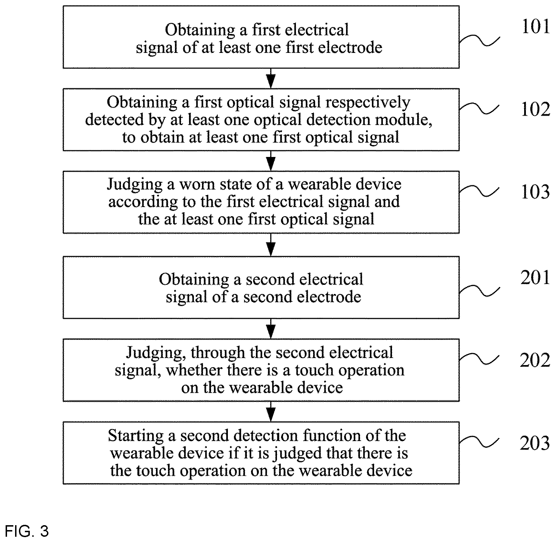

When detecting a physiological function of a user through the wearable device, for instance, when the user is subjected to electrocardiograph detection, the user is required to trigger the wearable device to start an electrocardiograph detection function. Based on this, in a possible implementation, FIG. 3 is a schematic flowchart of a wearing detection method provided by another embodiment of the present application. The method can be executed by a wearing detection apparatus, which can be implemented in the form of software and/or hardware. For instance, the apparatus may be part or the whole of a terminal device. The terminal device may be a wearable device, such as a heart rate bracelet, a heart rate earphone, a health bracelet, a health watch, a blood oxygen bracelet, a blood oxygen watch and other optical wearable devices; and the terminal device may also be a medical device, a fitness device, etc. The apparatus may also be a detection chip or others in the terminal device. The wearing detection method is described below by taking a terminal device as an executive entity. As shown in FIG. 3, after it is judged that the wearable device is in the worn state, the wearing detection method provided by the embodiment of the present application can further include:
Step S201: obtaining a second electrical signal of a second electrode.

The electrical signal of the second electrode can be obtained through the second electrode, and the second electrode may be one or more. The embodiment of the present application does not limit the quantity, location, structure of the second electrode.

Step S202: judging, through the second electrical signal, whether there is a touch operation on the wearable device.

The judging, through an electrode signal, whether there is the touch operation on the second electrode can be implemented by means of capacitance change, current change, voltage change and other changes of the electrode signal, which is not limited in the embodiment of the present application.

Step S203: starting a second detection function of the wearable device if it is judged that there is the touch operation on the wearable device.

The second detection function of the wearable device is started if it is judged that there is the touch operation on the wearable device. The specific function of the second detection function is not limited in the embodiment of the present application. In a possible implementation, the second detection function includes an electrocardiograph (ECG) detection function, so that the wearable device starts to perform the electrocardiograph detection for the user, which is not limited in the embodiment of the present application.

In the embodiment of the present application, it is judged whether there is the touch operation on the wearable device performed by the user by obtaining the second electrical signal of the second electrode, and the ECG detection function of the wearable device is started on the basis that it is judged that there is the touch operation on the wearable device, thereby improving the user experience.

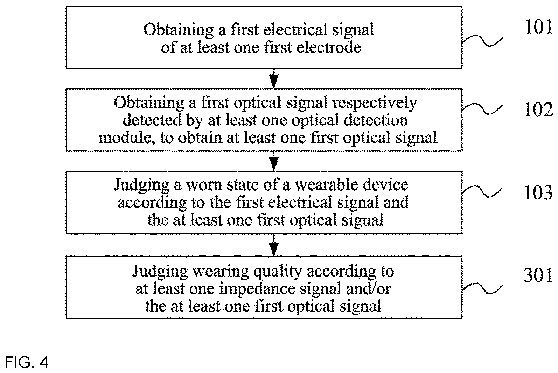

In a possible implementation, FIG. 4 is a schematic flowchart of a wearing detection method provided by yet another embodiment of the present application. The method can be executed by a wearing detection apparatus, and the apparatus may be implemented in the form of software and/or hardware. For instance, the apparatus may be part or the whole of a terminal device. The terminal device may be a wearable device, such as a heart rate bracelet, a heart rate earphone, a health bracelet, a health watch, a blood oxygen bracelet, a blood oxygen watch and other optical wearable devices; and the terminal device may also be a medical device, a fitness device, etc. The apparatus may also be a detection chip or others in the terminal device. The wearing detection method is described below by taking a terminal device as an executive entity. As shown in FIG. 4, after it is judged that the wearable device is in the worn state, the wearing detection method provided by the embodiment of the present application can further include:
Step S301: judging wearing quality according to the at least one impedance signal and/or the at least one first optical signal.

In a possible implementation, a magnitude of the impedance signal is in inverse proportion to the wearing quality. The wearing quality is judged according to at least one impedance signal, for instance, a plurality of preset ranges can be set, and each threshold range corresponds to a quality grade. For instance, three preset ranges are set for the impedance signal, and the three preset ranges respectively correspond to three grades: high quality, medium quality and low quality. The current grade of the wearing quality is judged by judging a preset range where the magnitude of the impedance signal is situated. If there are a plurality of impedance signals, the current grade of the wearing quality is judged through an average value of the plurality of impedance signals, or (not according to the claims) a quality grade corresponding to the most impedance signals is judged as the current grade of the wearing quality, the most impedance signals being situated within a certain preset range, or the like.

In a possible implementation, the quantity of the target optical signal of the at least one first optical signal is in direct proportion to the wearing quality. In a possible implementation, the judging of the wearing quality according to at least one first optical signal can be performed through the quantity or proportion of the target optical signal in the at least one first optical signal. According to the invention, all of the at least one first optical signal being the target optical signal indicates that the wearing quality in this case is relatively high, and the grade of the wearing quality is high quality; if the quantity of the target optical signal in the at least one first optical signal is smaller than a threshold quantity, or the proportion of the same is smaller than a preset threshold, it indicates that the wearing quality in this case is relatively inferior, and the grade of the wearing quality is low quality.

The judging of the wearing quality according to the at least one impedance signal and the at least one first optical signal is performed by combining the above two manners. When both the impedance signal and the first optical signal meet relatively high wearing quality, the wearing quality is judged to be relatively high; when both the impedance signal and the first optical signal meet relatively low wearing quality, the wearing quality is judged to be relatively low.

In another possible implementation, the wearing detection method provided by the embodiment of the present application can further include: judging the wearing quality to be inferior and/or reminding a user of inferior wearing quality, if a quantity of an impedance signal, which is within a third preset range, of the at least one impedance signal exceeds a third preset threshold, and/or a quantity of a first optical signal, which is within a fourth preset range, of the at least one first optical signal exceeds a fourth preset threshold.

By judging the quantity of the impedance signal, which is within the third preset range, of the at least one impedance signal and the quantity of the impedance signal, which is within the fourth preset range, of the at least one first optical signal, the wearing quality is judged to be inferior, and the user can also be reminded, thereby improving the user experience and ensuring the accuracy of detecting human physiological features by the wearable device. The wearing detection apparatus, chip, device, storage media and computer program product will be described below, and for their effects, the effects in the parts of the methods can be referred to, and will not be repeated hereinafter.

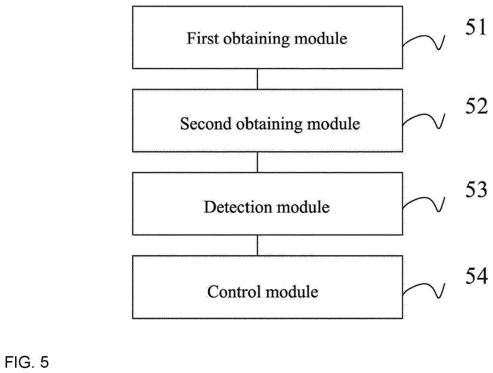

FIG. 5 is a schematic structural diagram of a wearing detection apparatus provided by an embodiment of the present application, and the apparatus may be implemented in the form of software and/or hardware. For instance, the apparatus may be part or the whole of a terminal device. The terminal device may be a wearable device, such as a heart rate bracelet, a heart rate earphone, a health bracelet, a health watch, a blood oxygen bracelet, a blood oxygen watch and other optical wearable devices; and the terminal device may also be a medical device, a fitness devices, etc. The apparatus may also be a detection chip or others in the terminal device. As shown in FIG. 5, the wearing detection apparatus provided by the embodiment of present application can include:
a first obtaining module 51, configured to obtain a first electrical signal of at least one first electrode;
a second obtaining module 52, configured to obtain a first optical signal respectively corresponding to at least one optical detection module, to obtain at least one first optical signal, where the optical detection module includes an optical transmitter and an optical receiver, and the first optical signal is an optical signal received by the optical receiver after being transmitted by the optical transmitter and going through a human body;
a detection module 53, configured to judge a worn state of a wearable device according to the first electrical signal and the at least one first optical signal.

Optionally, the first electrical signal includes at least one impedance signal, and the at least one impedance signal is an impedance signal between any two first electrodes of a plurality of first electrodes.

Optionally, the detection module 53 is specifically configured to:
determine an impedance signal, which is within a first preset range, of the at least one impedance signal to be a target impedance signal;
determine a first optical signal, which is within a second preset range, of the at least one first optical signal to be a target optical signal;
judge that the wearable device is in the worn state, if a quantity of the target impedance signal reaches a first preset threshold and a quantity of the target optical signal reaches a second preset threshold.

Optionally, the detection module 53 is specifically configured to:
determine an impedance signal, which is within a first preset range, of the at least one impedance signal to be a target impedance signal;
determine a first optical signal, which complies with a fluctuation rule of a human physiological signal, of the at least one first optical signal to be a target optical signal;
judge that the wearable device is in the worn state, if a quantity of the target impedance signal reaches a first preset threshold and a quantity of the target optical signal reaches a second preset threshold.

Optionally, the wearing detection apparatus provided by the embodiment of present application further includes a control module 54.

The control module 54 is configured to start a first detection function if it is judged that the wearable device is in the worn state, where the first detection function includes at least one of the following:
a heart rate detection function, a blood oxygen saturation detection function and a blood pressure detection function.

Optionally, the first obtaining module 51 is further configured to obtain a second electrical signal of a second electrode.

The detection module 53 is further configured to judge whether, through the second electrical signal, whether there is touch operation on the wearable device.

The control module 54 is further configured to start a second detection function of the wearable device if it is judged that there is the touch operation on the wearable device.

In a possible implementation, the second detection function includes an electrocardiograph (ECG) detection function.

Optionally, the control module 54 is further configured to: judge wearing quality according to the at least one impedance signal and/or the at least one first optical signal.

Optionally, a magnitude of the impedance signal is in inverse proportion to the wearing quality, and the quantity of target optical signal of the at least one first optical signal is in direct proportion to the wearing quality.

Optionally, the control module is further configured to:
judge the wearing quality to be inferior and/or remind a user of inferior wearing quality, if a quantity of the impedance signal, which is within a third preset range, of the at least one impedance signal exceeds a third preset threshold, and/or, if a quantity of a first optical signal, which is within a fourth preset range, of the at least one first optical signal exceeds a fourth preset threshold.

The apparatus embodiments provided by the present application are only for illustration. The module division in FIG. 5 is only a logical division, and there may be other forms of division in practical implementations. For instance, a plurality of modules may be combined or integrated into another system. A coupling of various modules may be realized through some interfaces. These interfaces usually are electrical communication interfaces, but it is not excluded that they may also be mechanical interfaces or interfaces in other forms. Therefore, the modules described as separate components may be or may not be physically separated, and may be located in one place, or distributed to different locations on the same device or different devices.

An embodiment of present application provides a chip, including:
at least one processor; and a memory in communicational connection with the at least one processor; where the memory stores instructions executable by the at least one processor, and the instructions are executed by the at least one processor to enable the at least one processor to execute the wearing detection methods provided by the above embodiments.

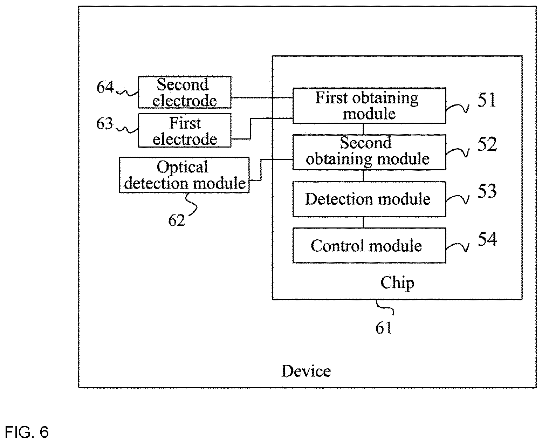

An embodiment of present application provides a device. FIG. 6 is a schematic structural diagram of a device provided by an embodiment of the present application. As shown in FIG. 6, the device provided by the embodiment of present application can include a chip 61.

Optionally, as shown in FIG. 6, the device provided by the embodiment of present application can further include: at least one optical detection module 62 and at least one first electrode 63, where the optical detection module 62 includes an optical transmitter and an optical receiver. The at least one first electrode 63, the optical transmitter and the optical receiver are located in a first plane of the device. The schematic structural diagram in FIG. 1 can be referred to. As shown in FIG. 1, the first electrode 111, the first electrode 112 and the optical detection module 120 are located in the first plane of the device.

Optionally, as shown in FIG. 6, the device provided by the embodiment of the present application can further include a second electrode 64, where the second electrode 64 is connected with the chip 61; the second electrode 64 is located in a second plane of the device; the first plane and the second plane are different planes; and the second electrode 64 is configured to judge whether there is a touch operation on the wearable device. The schematic structural diagram in FIG. 1 can be referred to. As shown in FIG. 1, the second electrode 113 is located in the second plane of the device, which is different from the plane in which the first electrode 111, the first electrode 112 and the optical detection module 120 are located.

The structure of the device is not limited thereto in the embodiment of the present application. Furthermore, an example not covered by the claims also provides a computer readable storage medium, where the computer readable storage medium stores computer executable instructions. When at least one processor of a user equipment executes the computer executable instructions, the user equipment executes the above various possible methods. The computer readable storage medium includes a computer storage medium and a communication medium. The communication medium includes any medium which facilitates a transfer of a computer program from one place to another. The storage medium may be any available medium that can be accessed by a general-purpose computer or a special-purpose computer. An exemplary storage medium is coupled to a processor, so as to enable the processor to read information from the storage medium and write information to the storage medium. Certainly, the storage medium may also be part of the processor. The processor and the storage medium may be located in an application specific integrated circuit (ASIC). Additionally, the ASIC may be located in the use equipment. Certainly, the processor and the storage medium may exist in a communication device as individual components.

## Claims

1. A wearing detection method, comprising:
obtaining (101) a first electrical signal of at least one first electrode;
obtaining (102) a first optical signal respectively detected by at least one optical detection module, to obtain at least one first optical signal, wherein the optical detection module comprises an optical transmitter and an optical receiver, and the first optical signal is an optical signal received by the optical receiver after being transmitted by the optical transmitter and going through a human body;
judging (103) a worn state of a wearable device according to the first electrical signal and the at least one first optical signal,
wherein the first electrical signal comprises at least one impedance signal, and the at least one impedance signal is an impedance signal between any two first electrodes of a plurality of first electrodes,
the judging (103) the worn state of the wearable device according to the first electrical signal and the at least one first optical signal comprises:
determining an impedance signal, which is within a first preset range, of the at least one impedance signal to be a target impedance signal;
determining a first optical signal, which is within a second preset range or complies with a fluctuation rule of a human physiological signal, of the at least one first optical signal to be a target optical signal; and
judging that the wearable device is in the worn state, if a quantity of the target impedance signal reaches a first preset threshold and a quantity of the target optical signal reaches a second preset threshold, wherein the first preset threshold is set according to a quantity of the first electrode and the second preset threshold is set according to a quantity of the optical detection module;
wherein the method further comprises:
judging (301) that a wearing quality is high if an average value of the at least one impedance signal is within a preset range corresponding to high quality grade and all of the at least one first optical signal are the target optical signal;
judging (301) that a wearing quality is low if an average value of the at least one impedance signal is within a preset range corresponding to low quality grade and the quantity of the target optical signal is less than a preset threshold corresponding to low quality grade.

2. The method according to claim 1, further comprising:
starting a first detection function if it is judged that the wearable device is in the worn state, wherein the first detection function comprises at least one of the following:
a heart rate detection function, a blood oxygen saturation detection function, and a blood pressure detection function.

3. The method according to claim 2, further comprising:
obtaining (201) a second electrical signal of a second electrode;
judging (202), through the second electrical signal, whether there is a touch operation on the wearable device;
starting (203) a second detection function of the wearable device if it is judged that there is the touch operation on the wearable device.

4. The method according to claim 3, wherein the second detection function comprises an electrocardiograph, ECG, detection function.

5. The method according to claim 1, further comprising:
a magnitude of the impedance signal is in inverse proportion to the wearing quality, and the quantity of the target optical signal of the at least one first optical signal is in direct proportion to the wearing quality.

6. The method according to claim 5, further comprising:
judging the wearing quality to be inferior and/or reminding a user of inferior wearing quality, if a quantity of an impedance signal, which is within a third preset range, of the at least one impedance signal exceeds a third preset threshold, and/or, if a quantity of a first optical signal, which is within a fourth preset range, of the at least one first optical signal exceeds a fourth preset threshold.

7. A chip (61), **characterised in that**, the chip comprises:
at least one processor; and
a memory in communicational connection with the at least one processor; wherein,
the memory stores instructions executable by the at least one processor, and the instructions are executed by the at least one processor to enable the at least one processor to execute the method according to any one of claims 1 to 6.

8. A device, **characterised in that**, the device comprises the chip (61) according to claim 7.

9. The device according to claim 8, further comprising: at least one optical detection module (120, 62) and at least one first electrode (111, 112, 63), wherein the at least one optical detection module (120, 62) and the at least one first electrode (111, 112, 63) are connected with the chip (61), the optical detection module (120, 62) comprises an optical transmitter and an optical receiver, and the at least one first electrode (111, 112, 63), the optical transmitter and the optical receiver are located in a first plane of the device.

10. The device according to claim 9, further comprising: a second electrode (113, 64), wherein the second electrode (113, 64) is connected with the chip (61); the second electrode (113, 64) is located in a second plane of the device; the first plane and the second plane are different planes; and the second electrode (113, 64) is configured to judge whether there is a touch operation on the wearable device.

## Patentansprüche

1. Verfahren zur Erkennung der Trageweise, umfassend:
Erfassung (101) eines ersten elektrischen Signals von mindestens einer ersten Elektrode;
Erfassung (102) eines ersten optischen Signals, das jeweils von mindestens einem optischen Erfassungsmodul erfasst wird, um mindestens ein erstes optisches Signal zu erhalten, wobei das optische Erfassungsmodul einen optischen Sender und einen optischen Empfänger umfasst und das erste optische Signal ein optisches Signal ist, das von dem optischen Empfänger empfangen wird, nachdem es von dem optischen Sender gesendet wurde und durch einen menschlichen Körper gegangen ist;
Beurteilung (103) eines Tragezustands einer tragbaren Vorrichtung anhand des ersten elektrischen Signals und des mindestens einen ersten optischen Signals,
wobei das erste elektrische Signal mindestens ein Impedanzsignal umfasst und das mindestens eine Impedanzsignal ein Impedanzsignal zwischen zwei beliebigen ersten Elektroden aus einer Vielzahl von ersten Elektroden ist,
die Beurteilung (103) des Tragezustands der tragbaren Vorrichtung gemäß dem ersten elektrischen Signal und dem mindestens einen ersten optischen Signal umfasst:
Bestimmung eines Impedanzsignals, das innerhalb eines ersten voreingestellten Bereichs liegt, des mindestens einen Impedanzsignals als Zielimpedanzsignal;
Bestimmung eines ersten optischen Signals, das innerhalb eines zweiten voreingestellten Bereichs liegt oder einer Fluktuationsregel eines humanphysiologischen Signals entspricht, des mindestens einen ersten optischen Signals als optisches Zielsignal; und
Beurteilung, dass sich die tragbare Vorrichtung im getragenen Zustand befindet, wenn eine Größe des Zielimpedanzsignals einen ersten voreingestellten Schwellenwert erreicht und eine Größe des optischen Zielsignals einen zweiten voreingestellten Schwellenwert erreicht, wobei der erste voreingestellte Schwellenwert gemäß einer Größe der ersten Elektrode und der zweite voreingestellte Schwellenwert gemäß einer Größe des optischen Erfassungsmoduls eingestellt wird;
wobei das Verfahren ferner Folgendes umfasst:
Beurteilung (301), dass eine hohe Tragequalität vorliegt, wenn ein Durchschnittswert des mindestens einen Impedanzsignals innerhalb eines voreingestellten Bereichs liegt, der einem hohen Qualitätsgrad entspricht, und alle der mindestens einen ersten optischen Signale das optische Zielsignal sind;
Beurteilung (301), dass eine Tragequalität niedrig ist, wenn ein Durchschnittswert des mindestens einen Impedanzsignals innerhalb eines voreingestellten Bereichs liegt, der einer niedrigen Qualitätsstufe entspricht, und die
Menge des optischen Zielsignals kleiner ist als ein voreingestellter Schwellenwert, der einer niedrigen Qualitätsstufe entspricht.

2. Verfahren nach Anspruch 1, ferner umfassend:
starten einer ersten Erkennungsfunktion, wenn beurteilt wird, dass sich die tragbare Vorrichtung im getragenen Zustand befindet, wobei die erste Erkennungsfunktion mindestens eine der folgenden Funktionen umfasst:
eine Funktion zur Erfassung der Herzfrequenz, eine Funktion zur Erfassung der Blutsauerstoffsättigung und eine Funktion zur Erfassung des Blutdrucks.

3. Verfahren nach Anspruch 2, ferner umfassend:
Erfassung (201) eines zweiten elektrischen Signals von einer zweiten Elektrode;
Beurteilung (202), durch das zweite elektrische Signal, ob eine Berührung des tragbaren Geräts vorliegt;
Starten (203) einer zweiten Erkennungsfunktion des tragbaren Geräts, wenn festgestellt wird, dass die Berührungsfunktion auf dem tragbaren Gerät vorhanden ist.

4. Verfahren nach Anspruch 3, wobei die zweite Erkennungsfunktion eine elektrokardiographische (EKG-) Erkennungsfunktion umfasst.

5. Verfahren nach Anspruch 1, ferner umfassend:
eine Größe des Impedanzsignals umgekehrt proportional zur Tragequalität ist und die Menge des optischen Zielsignals des mindestens einen ersten optischen Signals direkt proportional zur Tragequalität ist.

6. Verfahren nach Anspruch 5, ferner umfassend:
Beurteilung der Tragequalität als minderwertig und/oder Erinnern eines Benutzers an eine minderwertige Tragequalität, wenn eine Größe eines Impedanzsignals, das innerhalb eines dritten voreingestellten Bereichs liegt, des mindestens einen Impedanzsignals einen dritten voreingestellten Schwellenwert überschreitet, und/oder, wenn eine Größe eines ersten optischen Signals, das innerhalb eines vierten voreingestellten Bereichs liegt, des mindestens einen ersten optischen Signals einen vierten voreingestellten Schwellenwert überschreitet.

7. Ein Chip (61), **dadurch gekennzeichnet, dass** der Chip umfasst:
mindestens einen Prozessor; und
einen Speicher, der in Kommunikationsverbindung mit dem mindestens einen Prozessor steht; wobei,
der Speicher Anweisungen speichert, die von dem mindestens einen Prozessor ausgeführt werden können, und die Anweisungen von dem mindestens einen Prozessor ausgeführt werden, um den mindestens einen Prozessor in die Lage zu versetzen, das Verfahren nach einem der Ansprüche 1 bis 6 auszuführen.

8. Vorrichtung, **dadurch gekennzeichnet, dass** die Vorrichtung den Chip (61) nach Anspruch 7 umfasst.

9. Vorrichtung nach Anspruch 8, ferner umfassend: mindestens ein optisches Erfassungsmodul (120, 62) und mindestens eine erste Elektrode (111, 112, 63), wobei das mindestens eine optische Erfassungsmodul (120, 62) und die mindestens eine erste Elektrode (111, 112, 63) mit dem Chip (61) verbunden sind, das optische Erfassungsmodul (120, 62) einen optischen Sender und einen optischen Empfänger umfasst, und die mindestens eine erste Elektrode (111, 112, 63), der optische Sender und der optische Empfänger in einer ersten Ebene der Vorrichtung angeordnet sind.

10. Vorrichtung nach Anspruch 9, ferner umfassend: eine zweite Elektrode (113, 64), wobei die zweite Elektrode (113, 64) mit dem Chip (61) verbunden ist; die zweite Elektrode (113, 64) in einer zweiten Ebene der Vorrichtung angeordnet ist; die erste Ebene und die zweite Ebene unterschiedliche Ebenen sind; und die zweite Elektrode (113, 64) konfiguriert ist, um zu beurteilen, ob es eine Berührungsoperation auf der tragbaren Vorrichtung gibt.

## Revendications

1. Un procédé de détection de port, comprenant :
obtenir (101) un premier signal électrique d'au moins une première électrode ;
obtenir (102) un premier signal optique détecté, respectivement, par au moins un module de détection optique, pour obtenir au moins un premier signal optique, dans lequel le module de détection optique comprend un émetteur optique et un récepteur optique, et le premier signal optique est un signal optique reçu par le récepteur optique après avoir été transmis par l'émetteur optique et avoir traversé un corps humain ;
juger (103) de l'état de port d'un dispositif portable en fonction du premier signal électrique et d'au moins un premier signal optique,
dans lequel le premier signal électrique comprend au moins un signal d'impédance, et dans lequel ledit signal d'impédance est un signal d'impédance entre deux premières électrodes quelconques d'une pluralité de premières électrodes,
l'évaluation (103) de l'état de port du dispositif portable en fonction du premier signal électrique et d'au moins un premier signal optique comprend :
déterminer un signal d'impédance, qui se situe dans une première plage prédéfinie, d'au moins le signal d'impédance, comme étant un signal d'impédance cible ;
déterminer un premier signal optique, qui se situe dans une deuxième plage prédéfinie ou qui est conforme à une règle de fluctuation d'un signal physiologique humain, parmi au moins un premier signal optique, comme étant un signal optique cible ; et
juger que le dispositif portable est en état de port si une quantité du signal d'impédance cible atteint un premier seuil prédéfini, et qu'une quantité du signal optique cible atteint un deuxième seuil prédéfini, le premier seuil prédéfini étant fixé en fonction d'une quantité de la première électrode et le deuxième seuil prédéfini étant fixé en fonction d'une quantité du module de détection optique ;
dans lequel le procédé comprend en outre :
juger (301) qu'une qualité de port est élevée si une valeur moyenne d'au moins un signal d'impédance se situe dans une plage prédéfinie correspondant à un niveau de qualité élevé et si l'ensemble d'au moins un premier signal optique est le signal optique cible ;
juger (301) qu'une qualité de port est faible si une valeur moyenne d'au moins un signal d'impédance se situe dans une plage prédéfinie correspondant à un niveau de qualité faible et si la valeur moyenne d'au moins un signal d'impédance se situe dans une plage prédéfinie correspondant à un niveau de qualité faible
la quantité du signal optique cible est inférieure à un seuil prédéfini correspondant à un niveau de qualité inférieur.

2. Le procédé selon la revendication 1, comprenant en outre :
lancer une première fonction de détection s'il est jugé que le dispositif portable est en état de port, la première fonction de détection comprenant au moins l'un des éléments suivants :
une fonction de détection de la fréquence cardiaque, une fonction de détection de la saturation en oxygène du sang et une fonction de détection de la pression artérielle.

3. Le procédé selon la revendication 2, comprenant en outre :
obtenir (201) un deuxième signal électrique d'une deuxième électrode ;
juger (202), par le biais du deuxième signal électrique, s'il y a une opération tactile sur le dispositif portable ;
lancer (203) une deuxième fonction de détection du dispositif portable s'il est jugé qu'une opération tactile a lieu sur le dispositif portable.

4. Le procédé selon la revendication 3, dans laquelle la deuxième fonction de détection comprend une fonction de détection de l'électrocardiographe (ECG).

5. Le procédé selon la revendication 1, comprenant en outre :
l'amplitude du signal d'impédance est inversement proportionnelle à la qualité du port, et la quantité du signal optique cible d'au moins un premier signal optique est directement proportionnelle à la qualité du port.

6. La méthode selon la revendication 5, comprenant en outre :
juger que la qualité de port est inférieure et/ou rappeler à l'utilisateur que la qualité de port est inférieure, si la quantité d'un signal d'impédance, qui se situe dans une troisième plage prédéfinie, d'au moins un signal d'impédance dépasse un troisième seuil prédéfini, et/ou si la quantité d'un premier signal optique, qui se situe dans une quatrième plage prédéfinie, d'au moins un premier signal optique dépasse un quatrième seuil prédéfini.

7. Une puce (61), **caractérisée par le fait que** cette dernière comprend :
au moins un processeur ; et
une mémoire pouvant communiquer avec au moins un processeur ; dans laquelle,
la mémoire stocke des instructions exécutables par au moins un processeur, et dans laquelle les instructions sont exécutées par au moins un processeur pour permettre à au moins un processeur d'exécuter la méthode selon l'une des revendications 1 à 6.

8. Un dispositif, **caractérisé en ce qu'**il comprend la puce (61) selon la revendication 7.

9. Le dispositif selon la revendication 8, comprenant en outre : au moins un module de détection optique (120, 62) et au moins une première électrode (111, 112, 63), dans lequel au moins un module de détection optique (120, 62) et au moins une première électrode (111, 112, 63) sont connectés à la puce (61), le module de détection optique (120, 62) comprend un émetteur optique et un récepteur optique, et au moins une première électrode (111, 112, 63), l'émetteur optique et le récepteur optique sont situés dans un premier plan du dispositif.

10. Le dispositif selon la revendication 9, comprenant en outre : une deuxième électrode (113, 64), dans laquelle la deuxième électrode (113, 64) est connectée à la puce (61) ; la deuxième électrode (113, 64) est située dans un deuxième plan du dispositif ; le premier plan et le deuxième plan sont des plans différents ; et la deuxième électrode (113, 64) est configurée pour juger s'il y a une opération tactile sur le dispositif portable.
